# EUROPEAN PATENT APPLICATION

(11) **EP 0 632 041 A1**
(43) Date of publication of application: **04.01.1995**
(21) Application number: 93201929.2
(22) Date of filing: 01.07.1993
(51) Int. Cl.: C07D 489/00, A61K 31/485, C07D 489/02

(54) **New morphine derivatives having improved analgesic and narcotic properties**

(71) Applicant: KATHOLIEKE UNIVERSITEIT NIJMEGEN, NL-6500 HB Nijmegen (NL)
(72) Inventor: Dirksen, Ris, NL-6525 BS Nijmegen (NL)
(74) Representative: de Bruijn, Leendert C.

(57) **Abstract**

Morphine derivatives having the formula 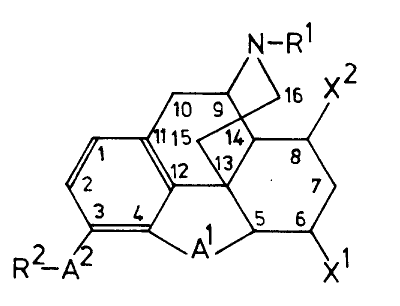
wherein:
A¹ represents -O-, -S-, -NR⁴- or -CHR⁵- ;
A² represents -O-, -S- or -NR⁴- ;
R¹ represents C₁-C₄ alkyl, C₁-C₄ alkenyl or cycloalkylmethyl;
R² represents H, C₁-C₆ alkyl, C₁-C₆ alkenyl, C₁-C₈ acyl or C₁-C₆ hydroxyalkyl;
one of X¹ and X² represents H and the other one is a group -A³-R³;
A³ represents -CH₂-, -CH₂CH₂-, -OCH₂-, -SCH₂-, -NR⁴CH₂-, -CO-, -NR⁴CO-, -SCO-, -SO₂-, -NR⁴SO₂-, -O-, -S- or -NR⁴-;
R³ represents an optionally substituted heterocyclic group;
R⁴ and R⁵ independently represent H or C₁-C₄ alkyl;
whereby the morphinan system may have a further unsaturation, in additon to the benzene ring.
These new morphine derivatives have improved analgesic and narcotic properties.

## Description

The present invention is in the pharmaceutical field. It relates to novel morphine derivatives. More in particular, the present invention relates to morphine derivatives having specific physicochemical properties which allow a pronounced narcotic-analgesic type of effect.

### State of the art

Morphine and codeine are the most important phenanthrene alkaloids of opium. Modification of the morphine molecule is a known method to change the pharmacological properties of morphine and codeine. Modifications that have been described and that resulted in molecules having analgesic properties include methyl substitution at the C-3 site (codeine), substitution at both C-3 and C-6 by acetyl (3,6-diacetyl-morphine = heroin), and by nicotinoyl (3,6-dinicotinoyl-morphine = nicomorphine, Vilan®). These structures lack the phenolic hydroxyl group which contributes strongly to the affinity for the opioid receptors [Reden J. et al, *J. Med. Chem.* **22**: 256 (1979); Lobbezoo M.W. et al. *Eur. J. Pharmacol*. **82**: 207-211 (1982)], and the low activity at the receptor level fails to account for the powerful analgesic effect of these compounds.

Subsequent studies have shown that the phenolic compounds are formed due to a rapid metabolic cleavage of the C-3 bond [Proceedings of the Vilan workshop, Graz 1987, Kopra H, Booy L.H.D.J. eds., 1988]. Analgesic activity is retained by the molecule when the 6-OH position is substituted by a nicotinoyl moiety [Koopman-Kimenai P.M. at al, *Pharm. Weekbl. Sci* **13** 142-147, (1991), *Eur. J. Clin. Pharmacol.* **41**: 375-378 (1991)]. When 3,6-dinicotinoylmorphine is administered to patients, 6-mononicotinoylmorphine, morphine and morphine-6-glucuronide are the active compounds. Some of the molecules that result from a metabolic conversion have a greater potency than that of parent compounds, as evidenced by the significantly lower ED₅₀. Thus, the effectiveness of the C-3 nicotinoyl-substituted morphine derivatives is explained by the effectiveness of the metabolic products rather than that of the parent substances. Substitutions at C-3 and C-6, however, result in physicochemical properties of the resulting molecules that differ from those of morphine, and such changes favour the transport into the central nervous system. There, too, active metabolites are formed. The lipid solubility of nicomorphine at a pH of 7.4 is about 10-fold higher than that of morphine, and this results in a lower ED₅₀. Moreover, the substitutions have been shown to enhance the safety of the drug in the specific application of perispinal opioid injections. Substituted molecules did not cause the high incidence of undesired side-effects, such as delayed respiratory depression [Dirksen, thesis, University of Nijmegen (1983)]. Also, a higher lipid solubility is believed to lower the toxicity of the drug after systemic use. However, the necessity of metabolic conversion, which differs among the individual physiological systems, reduces the predictability of the effect. Therefore, there is a need for morphine derivatives having tailored activities, which are not dependent on the metabolism of the compounds.

Recently it became evident that morphine-6-glucuronide (M-6-G) has the same analgesic activity as morphine itself. Although M-6-G is considered to be a highly polar conjugate, it can penetrate the blood-brain barrier and react with the receptor of analgesic action without prior hydrolysis of the glucuronide linkage [Yoshimura H. et al. *Biochem. Pharmacol.* **22**: 1423-1430 (1973)]. M-6-G binds to the µ-receptor. The agonist nature of M-6-G is considerably more potent in its antinociceptive properties than morphine in writhing (45 fold) and in tail-flick (61 fold) tests [Frances B. et al. *Progress Clin. Biol*. *Res.* **328**: 477-480 (1990)]. Its ability to displace ³H-naloxone from the homogenate of bovine caudate nucleus is comparable to that of morphine [Christensen C.B. et al. *Pharmacol. Toxicol.* **60**: 75-66 (1987)]. Glucuronides are generally considered as highly polar metabolites which are unable to cross the blood-brain barrier and are rapidly excreted by the urinary and/or biliary routes. M-6-G, and to a lesser extent M-3-G, are far more lipophilic than predicted and in fact not much less lipophilic than morphine itself [Carrupt P.A. et al*. J. Med. Chem.* **34**: 1272-1275 (1991)). Force-field and quantum mechanical calculations indicate that the two glucuronides can exist in conformational equilibrium between extended and folded forms. The extended conformers must be highly hydrophilic forms, because they efficiently expose their polar groups, predominating in polar media such as water. In contrast, the folded conformers mask part of their polar groups and are thus more lipophilic and likely to predominate in media of low polarity such as biological membranes.

M-3-G had poor affinity in all binding studies, whereas M-6-G effectively labelled µ, but not δ or κ receptors, with affinities similar to morphine. Microinjection of M-3-G directly into the periaquaductal grey were without effect. M-6-G, on the other hand, was up to 20-fold more potent than morphine following microinjection in the same region. High doses of M-6-G produced profound seizure activity. All 6-glucuronide actions were sensitive to the opiate antagonist naloxone [Pasternak G.W. et al. *Life Sci*. **41**: 2845-2849 (1987)].

Subcutaneous administration of M-6-G in mice elicited analgesia with an effect approximately twice that of morphine. When given intrathecally or intravenously, M-6-G was 90 and 650 fold, respectively, more potent as analgesic than morphine. While morphine was equipotent at both levels of the neuraxis as an analgesic, the 6β-glucuronide was approximately 5-fold more effective at the level of the spinal cord than supraspinally. The µ₁-selective antagonist naloxonazine blocked the analgesic effect of systemic and intracerebroventricular morphine-6B-glucuronide much as it blocked morphine, implying a role for µ₁-receptors in these actions. Like morphine, M-6β-G analgesia after intrathecal injection was not sensitive to naloxazine, suggesting a µ₂ mechanism within the spinal cord. These results imply that M-6β-G elicits its analgesic actions through the same receptor mechanisms as morphine [Paul D. et al. *J. Pharmacol. Exp. Ter.* **251**: 427-433 (1989); Dirksen R. *Pharm. Weekbl. Sci*. **12**: 41-45 1990)]. Osborne et al. [*Lancet* 1988:i; 828] investigated the analgesic effect of M-6-G in patients and found that the analgesic activity of morphine was due to the presence of the metabolite. M-3-G is thought to interfere with the antinociceptive effect and to inhibit the effects of morphine [Ekblom M. *Biopharm. Drug Dispos.* **14**: 1-11 (1993); Smith M.T. et al. *Life Sci.* **47P**: 579-585 (1990)]. Both glucuronides are eliminated from the body by renal clearance. Impaired kidney function leads to accumulation of both glucuronides and to prolonged half-life times [Hasselström J. et al. *Br. J. Clin. Pharmacol.* **27**: 515-518 (1989)].

### Summary of the invention

The present invention aims at providing new morphine derivatives according to two general types:
(a): These are selective opioid analgesics having an intermediate lipophilicity (i.e. a P7.4 in the range of 10 to 50), which have an intrinsic activity when interacting with the opioid receptors. These compounds are meant to be injected intrathecally (i.e. into the spinal subarachnoid space) or epidurally using techniques that are well-established in the clinical art. These compounds will be soluble in isotonic solutions and their solubilised form is compatible with nervous structures without inflicting damage to nervous or other types of structures.
(b): These are compounds producing useful narcotic effects in addition to their analgesic activity. They have a high lipophilicity (lipid solubility) and are designed for systemic (i.e. oral, intramuscular, intravenous, or suppositorial) administration.

The novel morphine derivatives of the present invention have the following formula: 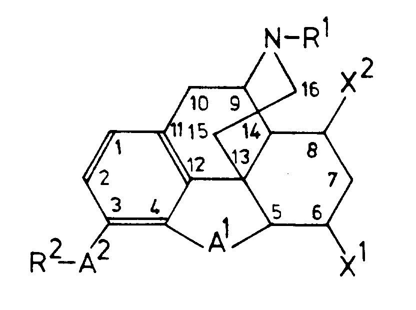
wherein:
A¹ represents -O-, -S-, -NR⁴- or -CHR⁵- ;
A² represents -O-, -S- or -NR⁴- ;
R¹ represents C₁-C₄ alkyl, C₁-C₄ alkenyl or cycloalkylmethyl;
R² represents H, C₁-C₆ alkyl, C₁-C₆ alkenyl, C₁-C₈ acyl or C₁-C₆ hydroxyalkyl;
one of X¹ and X² represents H and the other one is a group -A³-R³;
A³ represents -CH₂-, -CH₂CH₂-, -OCH₂-, -SCH₂-, -NR⁴CH₂-, -CO-, -NR⁴CO-, -SCO-, -SO₂-, -NR⁴SO₂-, -O-, -S- or -NR⁴-;
R³ represents an optionally substituted heterocyclic group;
R⁴ and R⁵ independently represent H or C₁-C₄ alkyl;
whereby one of the C-C bonds between positions 6 and 7, 7 and 8, and 8 and 14, or both of the C-C bonds between positions 6 and 7, and 8 and 14 of the morphinan system may be unsaturated. The term "C₁-C₈acyl" is intended to cover both aliphatic, aromatic and heterocylic acyl groups, optionally substituted, such as acetyl, lactoyl, benzoyl, phenylacetyl or nicotinoyl.

### Detailed description of the invention

The novel morphine derivatives according to the invention are compounds having the morphinane (1,2,3,9,10,10a-hexahydro-4*H*-10,4a-iminoethano-phenanthrene) system, or the corresponding 3,9,10,10a-tetrahydro analogue as in morphine, codeine and thebaine, or 1,9,10,10a-tetrahydro analogue as in pseudocodeine, or 2,3,9,10-tetrahydro analogue as in neopine, or 3,9-dihydro analogue as in oripavine. In the following, the conventional morphine ring numbering, which differs from the systematic numbering used above e.g. in that it starts at the benzene end ring rather than at the (semi)saturated end ring, will be adhered to.

The derivatives according to the invention have a one-atom bridge between carbon atoms 4 and 5 of the morphinane system, as in most natural morphine analogues. This bridge may be oxygen, sulphur, imino, alkylimino, methylene or alkylidene, preferably oxygen, sulphur, imino or methylene, most preferably oxygen.

The 3-position preferably bears a hydroxyl group, as in morphine, or else a methoxy group, as in codeine, pseudocodeine and thebaine, or a group which is susceptible to rapid metabolic cleavage, such as mercapto, alkylthio, amino, alkylamino, acylamino, alkoxy, alkanoyl, aroyl, or heterocyclic acyl, including nicotinoyl; alkyl represents herein lower alkyl, such as methyl, ethyl or isopropyl.

The 17-position (nitrogen atom) of the morphine derivatives of the invention can be substituted by a methyl group, as in morphine and codeine, or another lower alkyl or lower alkenyl group, especially allyl as in nalorphine, or cycloalkylmethyl as in cyclorphine (cyclopropyl) and nalbuphine (cyclobutyl). The preferred 17-substituent is methyl.

The derivatives according to the invention have a substituent at the 6-position, or alternatively at the 8-position, either in α- or in β-position, which is not readily metabolisable and provides a medium to high lipophilicity. More specifically, C-6 bears a heterocyclic group which is connected to the morphinan system by a poorly hydrolysable or non-hydrolysable group, as is mentioned above. Preferred connecting groups are aminocarbonyl, aminosulphonyl and thiocarbonyl, especially aminocarbonyl. The heterocyclic system may be substituted e.g. by lower alkyl, lower hydroxyalkyl, amino, lower alkylamino, hydroxy or lower alkoxy. The heterocycle preferably contains nitrogen, such as in pyridine, pyrimidine, pyrazine, triazole, pyrrole, imidazole, thiazole, oxazole, and their partially saturated and saturated analogues. A preferred heterocycle is pyridine, which is especially bound via its 3-position. Another preferred group of heterocycles comprises hydroxy substituted tetrahydrofuranyl or tetrahydropyranyl groups, as in monosaccharides (glycose and uronic acid residues). The invention does not comprise the known use of 6-α-1-glucuronides of morphine.

Whenever reference is made to lower alkyl or lower alkoxy, this term is to be understood as comprising any alkyl or alkoxy groups, unbranched or branched, having 1-6 carbon atoms, in particular 1-4 carbon atoms, such as methyl, ethyl, propyl, isopropyl and the butyl isomers. The preferred lower alkyl group is generally methyl.

The compounds according to the invention can be prepared along synthetic pathways which are known per se. Some non-limiting possibilities are given below.

### 6-Acylamino derivatives:

- Starting with codeine, the C7-C8 double bond is catalytically hydrogenated, whereafter the 6-OH group is oxidized, e.g. using benzophenone, and the resulting ketone is subjected to reductive amination, e.g. with sodium cyanoborohydride. The 6-amino compound is acylated with a substituted heterocyclic group and, if desired, the aromatic methoxy group is converted to a hydroxy group e.g. using boron tribromide.
- Starting with codeine, the 6-OH group is converted to the p-toluenesulfonate, which is reacted with sodium azide to the 6-azido compound. Catalytic hydrogenation of the azido group results in a 6-amino-7,8-dihydro-codeine derivative, which can be converted to the desired final product as indicated above.
- Starting with morphine, the same synthetic routes are set out above can be followed, resulting in the corresponding morphine derivatives. Similarly the 8-acylamino compounds can be prepared starting with pseudocodeine using the routes outlined above.

### 6-Acylthio derivatives:

- Starting with codeine, the 6-OH group is converted to the p-toluenesulphonate, which is reacted with potassium hydrosulphide to the 6-mercapto compound. The latter is acylated with a substituted heterocyclic group and, if desired, the aromatic methoxy group is converted to a hydroxy group e.g. using boron tribromide.
- Starting with codeine, the C7-C8 double bond is catalytically hydrogenated, whereafter the 6-OH group is converted to the 6-bromide via the Mitsonobu reaction. The 6-bromo compound is reacted with thioureum and sodium hydroxide to the 6-mercapto compound, which can be reacted further as described above.

### 6-Glucuronido-amino derivatives:

- Starting with the 6-amino derivative of 7,8-dihydrocodeine, which can be obtained as described above, the amino group is acetylated, and then reacted with trimethylchlorosilane to produce the trimethylsilylamide which is subsequently reacted with methyl (2,3,4-tri-*O*-acetyl-D-glucopyran)uronate and trimethylsilyl trifluoromethanesulphonate as a catalyst. After deprotection of the acetylated glucuronide with sodium methoxide, the aromatic methoxy group can be converted to a hydroxy group e.g. using boron tribromide.

### 6-Glucuronido-thio derivatives:

- Starting with the 6-mercapto derivative of 7,8-dihydrocodeine, which can be obtained as described above, the mercapro group is coupled to α-bromomethyl-(2,3,4-tri-*O*-acetyl-D-glucopyran)uronate using silver carbonate as a catalyst. After deprotection of the acetylated glucuronide with sodium methoxide, the aromatic methoxy group can be converted to a hydroxy group e.g. using boron tribromide.

Other 6-sugar derivatives can be synthesizecd in similar way.

The invention also relates to pharmaceutical compositions containing a morphine derivative as described above. The pharmaceutical compositions of the invention further contain a carrier in accordance with the intended administration form (e.g. oral, intrathecal, intravenous, epidural, etc.), as well as further optional adjuvants, stabilizers, and other optional auxiliaries which are known in the art.

### Example I

### Synthesis of 3-hydroxy-17-methyl-6,α-nicotinoylamino-morphinan.

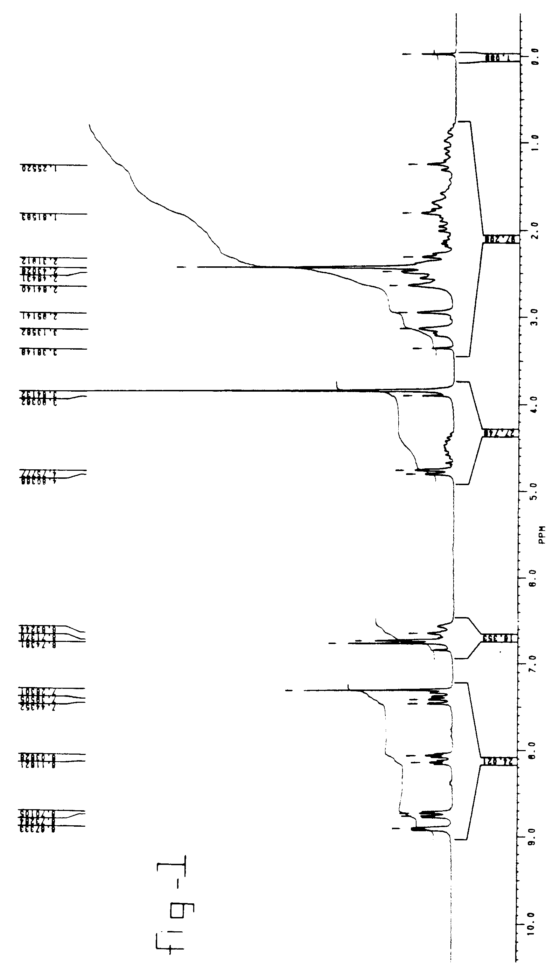

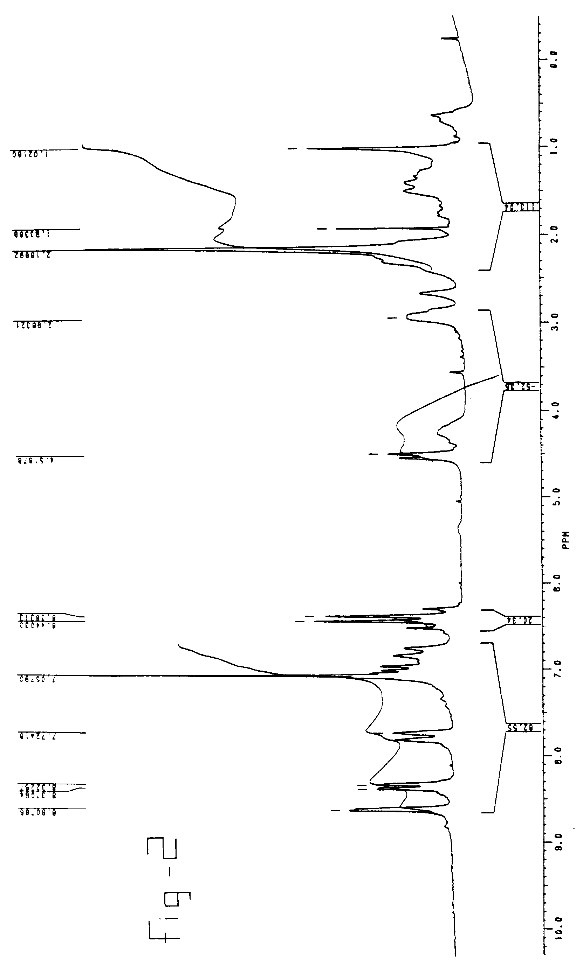

(a) Synthesis of dihydrocodeine
   Codeine (5.04 g, 13.5 mmoles) was suspended in 50 ml of water. To the suspension sufficient glacial acetic acid was added to dissolve all solids. 1 g of 5% Pd/BaSO₄ was added and the solution was hydrogenated at room temperature. The catalyst was filtered off after 3 hours and was washed twice with warm water. The pH of the filtrate was adjusted to 12 using 6 m KOH and the filtrate was then extracted three times with 200 ml chloroform/ether (1/1). The combined organic layers were washed with a saturated NaCl solution, dried on MgSO₄ and evaporated. The oily residue solidified after some time.
   Yield: 3.5 g (86%).
   M.p.: 109-110°C.
b) Synthesis of dihydrocodeinone
   Potassium t-butoxide (3.81 g, 34 mmoles) was dissolved in 400 ml of refluxing dry benzene in a 1 l three-necked flask fitted with a condenser, a thermometer and a nitrogen inlet. When the solution was almost clear, a solution of dihydrocodeine (3.4 g, 11.4 mmoles) and benzophenone (20.6 g, 114 mmoles) in 300 ml of dry benzene was added. After refluxing for 2.5 hours, the solution was cooled to 4°C. 20 ml of 1.5 M HCl were added, the benzene layer was separated and extracted three times with 22 ml 1.5 M HCl. The combined aqueous layers were washed once with 50 ml of ether, and the pH was then adjusted to 12 using concentrated NaOH. The alkaline aqueous layer was extracted five times with 50 ml of ethyl acetate. The extract was dried on MgSO₄, treated with activated carbon and evaporated.
   Yield: 2.65 g (78%);
   M.p.: 196-198°C.
c) Synthesis of 7,8-dihydro-6(α,β)amino-codeine
   A solution of sodium cyanoborohydride (0.21 g, 3.4 mmoles) in 10 ml of methanol was added under nitrogen to a solution of dihydrocodeinone (1.6 g; 5.4 mmoles) and ammonium acetate (4.16 g, 54 mmoles) in 30 ml of methanol. The pH was adjusted to 7 using concentrated HCl. After stirring for 24 hours at room temperature, the pH of the solution was adjusted to 1 with HCl. The solution was then evaporated to dryness. The residue was dissolved in 20 ml of water and extracted three times with chloroform. The solution was adjusted to pH 13, saturated with NaCl and extracted three times with chloroform. The combined chloroform extracts were dried on MgSO₄, treated with activated carbon and evaporated in vacuo. The resulting oil was not purified further.
   Yield: 1.5 g (90%).
d) Synthesis of 7,8-dihydro-6(α,β)-nicotinoylaminocodeine
   To a solution of the 7,8-dihydro-6-amino-codeine prepared under c) (1.5 g, 5 mmoles) and triethylamine (1.21 g, 12 mmoles) in 15 ml of dichloromethane at 4°C was added nicotinoylchloride.HCl (1 g, 5.6 mmoles) portionwise. De resulting suspension was stirred at 4°C for 3 hours and then at room temperature overnight. The suspension was evaporated to dryness and the residue was dissolved in 20 ml of water. The solution was acidified to pH 1 and extracted three times with chloroform. The water layer was adjusted to pH 13 using solid KOH and extracted three times with chloroform. The combined chloroform layers were dried on Na₂SO₄ and then evaporated to dryness. The residue was subjected to chromatography (silica 60, eluent chloroform/methanol 17/3). About 300 mg of pure 6α-nicotinoyl compound was isolated. The structure was elucidated by means of the NMR spectrum. The coupling between H-4 and H-5 is ± 4 Hz, indicating that H4 and H5 are in a cis relationship, i.e. the isolated compound is the 6α-isomer. ¹H-NMR (CDCl₃): see Figure 1
e) Synthesis of 7,8-dihydro-6α-nicotinoylaminomorphine
   The 7,8-dihydro-6α-nicotylaminocodeine prepared under d) (160 mg, 0.4 mmoles) in 1 ml of chloroform was added dropwise to a solution of boron tribromide (2.4 ml of 1 M solution in dichloromethane diluted with chloroform to 7 ml). After stirring at room temperature for two hours, the suspension was poured into 8 ml of ice and 2 ml of ammonia solution. After stirring at 0°C for 15 minutes, the phases were separated. The water layer was extracted once with chloroform. The combined chloroform layers were dried on NaSO₄, treated with activated carbon and evaporated to dryness. Chromatography (silica 60, eluent chloroform/methanol 85/15) of the residue yielded 60 mg (45%) of the morphine compound.
   [α]_{D}²² = -180°C (c = 1.4 chloroform). ¹H NMR (CDCl₃): see Figure 2.

Pharmacologic activity of 7,8-dihydro-6α-nicotinoylaminocodeine and 7,8-dihydro-6α-nicotinoylaminomorphine:
Intravenous and intrathecal injection of these compounds in animal experiments using rats showed a strong reflex inhibitory potency of the compounds. After intrathecal injection, a long-lasting antinociceptive efficacy was observed.

## Claims

1. A morphine derivative having the formula 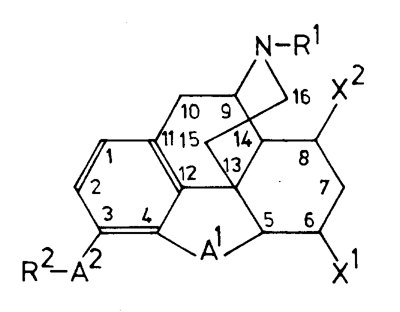 wherein:
A¹ represents -O-, -S-, -NR⁴- or -CHR⁵- ;
A² represents -O-, -S- or -NR⁴- ;
R¹ represents C₁-C₄ alkyl, C₁-C₄ alkenyl or cycloalkylmethyl;
R² represents H, C₁-C₆ alkyl, C₁-C₆ alkenyl, C₁-C₈ acyl or C₁-C₆ hydroxyalkyl;
one of X¹ and X² represents H and the other one is a group -A³-R³;
A³ represents -CH₂-, -CH₂CH₂-, -OCH₂-, -SCH₂-, -NR⁴CH₂-, -CO-, -NR⁴CO-, -SCO-, -SO₂-, -NR⁴SO₂-, -O-, -S- or -NR⁴-;
R³ represents an optionally substituted heterocyclic group;
R⁴ and R⁵ independently represent H or C₁-C₄ alkyl;
whereby one of the C-C bonds between positions 6 and 7, and 7 and 8, or both of the C-C bonds between positions 6 and 7, and 8 and 14 of the morphinan system may be unsaturated.

2. A morphine derivative according to claim 1, wherein A¹ represents -O-.

3. A morphine derivative according to claim 1, wherein A² represents -O-.

4. A morphine derivative according to claim 1, wherein R¹ represents methyl.

5. A morphine derivative according to claim 1, wherein R² represents H.

6. A morphine derivative according to claim 1, wherein X² represents H.

7. A morphine derivative according to claim 1, wherein A³ represents -NR⁴CO- or -SCO-, in particular -NHCO-.

8. A morphine derivative according to claim 1, wherein R³ represents a nitrogen-containing heterocycle, in particular 3-pyridyl.

9. A morphine derivative according to claim 1, wherein R³ represents a glycose or glyconic acid residue, with the exception of an α-1-glucuronide.

10. A pharmaceutical composition containing a morphine derivative according to one of the preceding claims.

11. Use of a morphine derivative according to one of the preceding claims for preparing a pharmaceutical composition having analgesic and/or narcotic properties.
